# EUROPEAN PATENT SPECIFICATION

(11) **EP 4 494 619 B1**
(45) Date of publication and mention of the grant of the patent: **05.11.2025**
(21) Application number: 23803370.8
(22) Date of filing: 19.04.2023
(51) Int. Cl.: A61F 13/15, A61F 13/49

(54) **METHOD AND DEVICE FOR MANUFACTURING DISPOSABLE WEARABLE ARTICLE**
VERFAHREN UND VORRICHTUNG ZUR HERSTELLUNG EINES WEARABLE-EINWEGARTIKELS
PROCÉDÉ ET DISPOSITIF DE FABRICATION D'UN ARTICLE PORTABLE JETABLE

(30) Priority: 13.05.2022 JP 2022079835
(43) Date of publication of application: 22.01.2025
(73) Proprietor: Zuiko Corporation, Ibaraki-shi, Osaka 567-0082 (JP)
(72) Inventor: YAMAUCHI Hirofumi, Ibaraki-shi, Osaka 567-0082 (JP)
(74) Representative: Hasegawa, Kan
(86) International application number: PCT/JP2023/015682
(87) International publication number: WO 2023/218888

(56) References cited:
- WO-A1-2014/176007
- WO-A1-2014/176007
- WO-A1-2019/142691
- WO-A1-2019/142691
- JP-B1- 6 903 810

## Description

### TECHNICAL FIELD

The present invention relates to a method and a device for manufacturing a disposable wearable article, and more specifically, relates to a technique for manufacturing a substantially T-shaped disposable wearable article.

### BACKGROUND ART

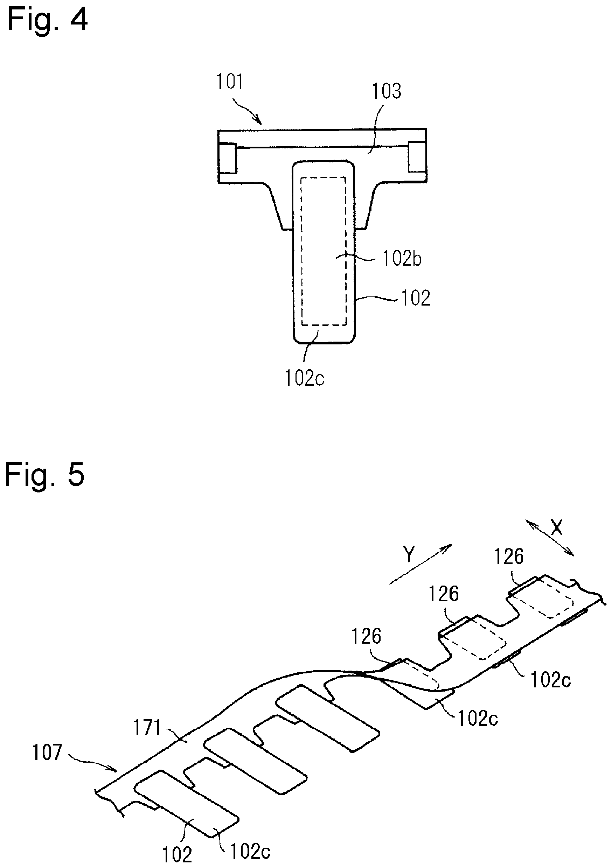

For example, Fig. 4 is a developed view of a substantially T-shaped disposable wearable article 101. As shown in Fig. 4, in the disposable wearable article 101, one end side of an absorbent body 102 is joined to an intermediate position of a waist member 103. The disposable wearable article 101 is provided in a state where the other end side 102c of the absorbent body 102 is folded back at a central part 102b toward the waist member 103 so that the absorbent body 102 is folded in half and the waist member is folded so that both end parts overlap.

Fig. 5 is a perspective view showing a manufacturing process of the disposable wearable article 101. As shown in Fig. 5, a wearable article continuous body 107 in which a plurality of absorbent bodies 102 have one end sides thereof joined in a comb shape with a predetermined spacing provided therebetween is formed while a waist member continuous body 171 in which first sections to be made into waist members 103 are connected is being conveyed in the direction indicated by the arrow Y. Then, while the wearable article continuous body 107 is being conveyed, the waist member continuous body 171 side of the wearable article continuous body 107 is lifted and placed on the other end sides 102c of the absorbent bodies 102 to fold the absorbent bodies 102 in half so that fold lines 126 are formed at intermediate positions of the absorbent bodies 102 in a direction X orthogonal to the conveyance direction Y. Then, the wearable article continuous body 107 is cut between the adjacent absorbent bodies 102 to form disposable wearable article pieces (for example, see Patent Literature 1).

Patent Literature 2 discloses a folding device that successively cuts an absorbent body continuous body in which second portions to be made into absorbent bodies are connected, to separate the absorbent bodies while conveying it along a cylindrical conveyance route, and further, folds the absorbent bodies in half while conveying them along the cylindrical conveyance route.

### CITATION LIST

### PATENT LITERATURES

[Patent Literature 1] JP 4920424 B2
[Patent Literature 2] JP 2851784 B2
WO 2019/142691 A1 and
WO 2014/176007 A1 represent relevant prior art

### SUMMARY OF THE INVENTION

### PROBLEM TO BE SOLVED BY THE INVENTION

In a case where the absorbent bodies of the wearable article continuous body are folded in half while the wearable article continuous body is being conveyed, it is necessary to fold the absorbent bodies 102 in half with the angle being gradually increased so that no excessive force acts on the absorbent bodies 102. For this reason, the section for folding the absorbent bodies 102 in half is long, which increases the installation space of the disposable wearable article manufacturing device.

In view of such circumstances, a problem to be solved by the present invention is to provide a method and a device for manufacturing a disposable wearable article that can shorten the section for folding the absorbent bodies in half.

### MEANS FOR SOLVING THE PROBLEM

To solve the above-mentioned problem, the present invention provides a disposable wearable article manufacturing method structured as follows:

The disposable wearable article manufacturing method is provided with: (i) a first conveying step of conveying, in a first direction, an absorbent body continuous body in which first sections to be made into absorbent bodies are connected in the first direction; (ii) a second conveying step of conveying, in a second direction, a waist member continuous body in which second sections to be made into waist members are connected in the second direction; (iii) a first cutting step of forming the absorbent bodies by successively cutting the absorbent body continuous body being conveyed in the first direction; (iv) a turning step of turning the absorbent bodies 90 degrees; (v) a joining step of forming a wearable article continuous body by successively placing first regions on one end side of the 90-degree turned absorbent bodies on the waist member continuous body being conveyed in the second direction with a spacing provided therebetween such that second regions on the other end side of the absorbent bodies protrude from the waist member continuous body, and joining the first regions of the absorbent bodies to the waist member continuous body; (vi) a folding step of folding the absorbent bodies in half while the wearable article continuous body is being conveyed; and (vii) a second cutting step of cutting the waist member continuous body of the wearable article continuous body at cutting positions between the adjacent absorbent bodies while the wearable article continuous body is being conveyed. At the folding step, the absorbent bodies are folded in half by placing the second regions of the absorbent bodies on the first regions of the absorbent bodies while the wearable article continuous body is being conveyed along a cylindrical conveyance route.

According to the above-described method, disposable wearable articles can be manufactured in each of which the first region of the folded absorbent body is joined to an intermediate position of the waist member. Since the absorbent bodies are folded in half while the wearable article continuous body is being conveyed along the cylindrical conveyance route, the section for folding the absorbent bodies in half can be made short compared with when the absorbent bodies are folded in half while the wearable article continuous body is being conveyed along a linear conveyance route.

Preferably, the disposable wearable article manufacturing method is further provided with (viii) a temporarily fixing step of temporarily fixing the first regions and the second regions of the folded absorbent bodies together.

In this case, since the folded state of the absorbent bodies is stabilized, the wearable article continuous body to which the folded absorbent bodies are joined can be conveyed at higher speed, so that the number of disposable wearable articles manufactured per unit time can be increased.

Preferably, the disposable wearable article manufacturing method is further provided with (ix) a repitching step of, by using a moving pad, receiving the 90-degree turned absorbent bodies from the turning step, and transferring the received absorbent bodies to the joining step, the moving pad receiving the 90-degree turned absorbent bodies from the turning step while moving at a first speed, and transferring the received absorbent bodies to the joining step while moving at a second speed different from the first speed.

In this case, the spacing between the absorbent bodies placed on the waist member continuous body at the joining step can be changed.

Preferably, the disposable wearable article manufacturing method is further provided with (x) a size changing step of changing, according to the size of the disposable wearable articles, the spacing between the absorbent bodies placed on the waist member continuous body at the joining step by changing one or both of the conveyance speeds of the waist member continuous body and the absorbent body continuous body.

In this case, it is possible to accommodate size differences of the disposable wearable articles.

Moreover, to solve the above-mentioned problem, the present invention provides a disposable wearable article manufacturing device structured as follows:

The disposable wearable article manufacturing device is provided with: (a) a first unit that forms absorbent bodies by cutting an absorbent body continuous body in which first sections to be made into the absorbent bodies are connected in a first direction while conveying the absorbent body continuous body in the first direction, and turns the absorbent bodies 90 degrees; (b) a second unit that forms a wearable article continuous body by conveying, in a second direction, a waist member continuous body in which second sections to be made into waist members are connected in the second direction, successively placing first regions on one end side of the 90-degree turned absorbent bodies on the waist member continuous body being conveyed with a spacing provided therebetween such that second regions on the other end side of the absorbent bodies protrude from the waist member continuous body, and joining the first regions of the absorbent bodies to the waist member continuous body; and (c) a third unit that folds the absorbent bodies of the wearable article continuous body in half and cuts the wearable article continuous body at cutting positions between the adjacent absorbent bodies to form disposable wearable articles in each of which the first region of the folded absorbent body is joined to an intermediate position of the waist member. The third unit has (c-1) a winding portion that moves along a cylindrical conveyance route; and (c-2) a swinging pad that swings between an opened position adjacent to the winding portion in an axial direction of the conveyance route along the conveyance route and a closed position facing the winding portion in a radial direction of the conveyance route with the conveyance route in between, and moves along the conveyance route together with the winding portion, (c-1) when the swinging pad is at the opened position, the winding portion starts to hold the waist member continuous body of the wearable article continuous body and the swinging pad starts to hold the second region of the absorbent body of the wearable article continuous body, and (c-ii) while moving along the conveyance route, the swinging pad swings to the closed position to place the second region of the absorbent body on the first region of the absorbent body, thereby folding the absorbent body in half.

According to the above-described structure, since the absorbent bodies of the wearable article continuous body are folded in half while the wearable article continuous body is being conveyed along the cylindrical conveyance route, the section for folding the absorbent bodies in half can be made short compared with when the absorbent bodies are folded in half while the wearable article continuous body is being conveyed along a linear conveyance route.

Preferably, the disposable wearable article manufacturing device is further provided with (d) a temporarily fixing device that temporarily fixes the first regions and the second regions of the folded absorbent bodies together.

In this case, since the folded state of the absorbent bodies is stabilized, the wearable article continuous body to which the folded absorbent bodies are joined can be conveyed at higher speed, so that the number of disposable wearable articles manufactured per unit time can be increased.

Preferably, the disposable wearable article manufacturing device is further provided with (e) a repitching device having a moving pad that receives the 90-degree turned absorbent bodies from the first unit while moving at a first speed and transfers the received absorbent bodies to the second unit while moving at a second speed different from the first speed.

In this case, the spacing between the absorbent bodies placed on the waist member continuous body at the second unit can be changed.

Preferably, the disposable wearable article manufacturing device is further provided with (f) a size changing device that changes, according to the size of the disposable wearable articles, the spacing between the absorbent bodies placed on the waist member continuous body by changing one or both of a first conveyance speed at which the first unit conveys the absorbent body continuous body and a second conveyance speed at which the second unit and the winding portion of the third unit convey the waist member continuous body.

In this case, it is possible to accommodate size differences of the disposable wearable articles.

### EFFECTS OF THE INVENTION

According to the present invention, the section for folding the absorbent bodies in half can be shortened.

### BRIEF DESCRIPTION OF THE DRAWINGS

[Fig. 1] Fig. 1 is a schematic view of a disposable wearable article manufacturing device (first embodiment).
[Fig. 2] Fig. 2 is a schematic view of the disposable wearable article manufacturing device (first embodiment).
[Fig. 3] Fig. 3 is an explanatory view of a disposable wearable article manufacturing process (first embodiment).
[Fig. 4] Fig. 4 is a developed view of the disposable wearable article (first conventional example).
[Fig. 5] Fig. 5 is a perspective view showing the disposable wearable article manufacturing process (first conventional example).

### MODE FOR CARRYING OUT THE INVENTION

Hereinafter, an embodiment of the present invention will be described with reference to the drawings.

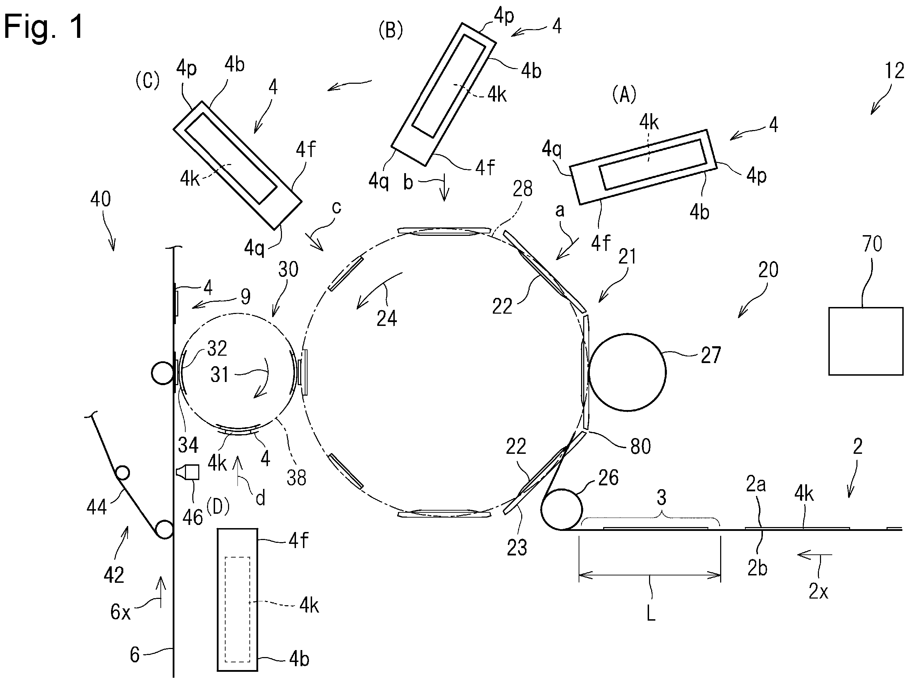

[First embodiment] A disposable wearable article manufacturing method and device of a first embodiment will be described with reference to Figs. 1 to 3.

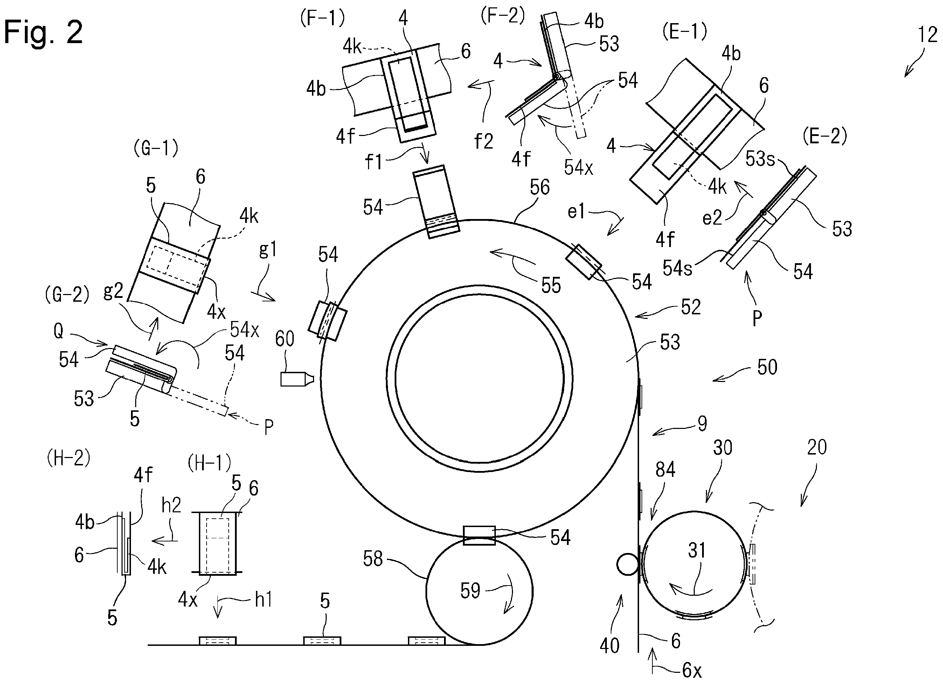

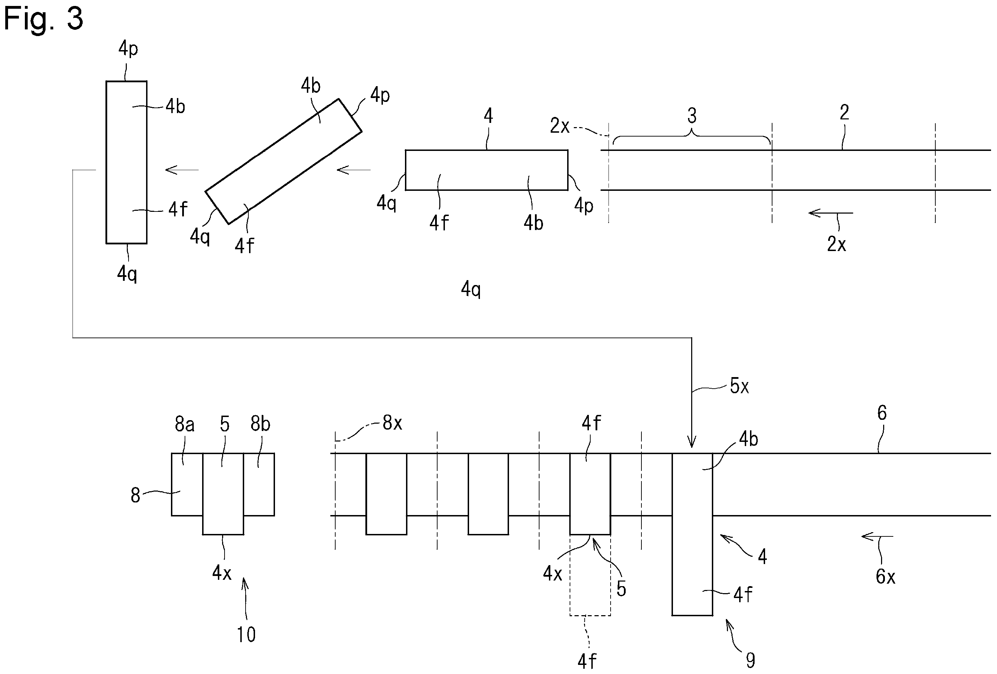

Figs. 1 and 2 are schematic views of a disposable wearable article manufacturing device 12. In Figs. 1 and 2, (A), (B), (C), (D), (E-1), (E-2), (F-1), (F-2), (G-1), (G-2), (H-1) and (H-2) are partial schematic views from the directions indicated by the arrows a, b, c, d, e1, e2, f1, f2, g1, g2, h1 and h2, respectively. Fig. 3 is an explanatory view showing a disposable wearable article manufacturing process.

As shown in Figs. 1 and 2, the disposable wearable article manufacturing device 12 is provided with a first unit 20, a repitching device 30, a second unit 40, a third unit 50, a temporarily fixing device 60 and a size changing device 70.

In the first unit 20, a guide roll 26 and a cutter roll 27 are disposed adjacent to a turning drum 21.

The turning drum 21 is provided with a plurality of suction tables 22 that move in a direction indicated by the arrow 24 along a cylindrical conveyance route 28. The suction tables 22 each hold an absorbent body continuous body 2 fed through the guide roll 26 by sucking it to a suction surface 23 where non-illustrated suction holes communicating with a negative pressure source are formed.

The cutter roll 27 forms absorbent bodies 4 by cutting the absorbent body continuous body 2 conveyed being sucked to the suction surfaces 23 of the suction tables 22, between the adjacent suction tables 22 at an appropriate timing. The suction tables 22 each move along the cylindrical conveyance route 28 while holding the absorbent body 4 on the suction surface 23, and turn 90 degrees, so that the direction connecting both ends 4p and 4q of the absorbent body 4 becomes orthogonal to the conveyance direction of the absorbent body 4.

In the absorbent body continuous body 2, first sections 3 having a length L and to be made into the absorbent bodies 4 are connected in a first direction 2x indicated by the arrow 2x. For example, in the absorbent body continuous body 2, absorbent cores 4k containing pulp and/or superabsorbent polymer or the like are arranged in the first direction 2x with a spacing provided therebetween between two belt-form sheets 2a and 2b. The absorbent body continuous body 2 is cut between the adjacent absorbent cores 4k, thereby forming the absorbent bodies 4.

The repitching unit 30 has a moving pad 32 that moves along a cylindrical conveyance route 38 in a direction indicated by the arrow 31 while changing the speed. The moving pad 32 has a suction surface 34 where non-illustrated suction holes communicating with a negative pressure source are formed. While moving at a first speed, the moving pad 32 sucks, to the suction surface 34, the 90-degree turned absorbent bodies 4 held by the suction table 22 of the turning drum 21 of the first unit 20, receives the 90-degree turned absorbent bodies 4, moves while changing the speed to a second speed different from a first speed, and transfers the received absorbent bodies 4 to the second unit 40 at the second speed. Using a servo motor makes it easy to change the speed of the moving pad 32.

The second unit 40 has a conveyer 42 disposed adjacent to the repitching device 30 and a joining device 46 disposed along the conveyer 42.

The conveyer 42 conveys, in a second direction 6x, a waist member continuous body 6 in which second sections 7 (see Fig. 3) to be made into waist members 8 (see Fig. 3) are connected in the second direction 6x. First regions 4b on one end 4p side of the 90-degree turned absorbent bodies 4 successively fed from the repitching device 30 are successively placed on the waist member continuous body 6 being conveyed by the conveyer 42, with a spacing provided therebetween such that second regions 4f on the other end 4q side of the absorbent bodies 4 protrude from the waist member continuous body 6. The joining device 46 joins the first regions 4b of the absorbent bodies 4 and the waist member continuous body 6 together, thereby forming a wearable article continuous body 9 in which the first regions 4b of the absorbent bodies 4 and the waist member continuous body 6 are joined together.

The conveyer 42 is provided with, for example, an endless belt 44 having air permeability and circulating along a predetermined route and a non-illustrated suction box disposed inside the endless belt 44 and connected to a negative pressure source, and is structured so as to convey the waist member continuous body 6 and the 90-degree turned absorbent bodies 4 while sucking them to the outside of the endless belt 44.

The joining device 46 previously applies an adhesive to at least one of a pair of facing parts of the waist member continuous body 6 and the absorbent bodies 4 which parts are to be placed one on another. The joining device 46 may be structured so as to join the absorbent bodies 4 and the waist member continuous body 6 together by heat sealing, ultrasonic wave sealing or the like instead of an adhesive.

While the repitching device 30 may be omitted, the provision of the repitching device 30 between the first unit 20 and the second unit 40 makes it possible to change the spacing between absorbent bodies 5 placed on the waist member continuous body 6 at the second unit 40.

The third unit 50 includes, as shown in Fig. 2, a folding drum 52, a guide roll 58, and a conveyer and a cutter which are not shown, folds the absorbent bodies 4 in half, and cuts the wearable article continuous body 9 at cutting positions between the adjacent absorbent bodies 4 or 5, thereby forming disposable wearable articles 10 in each of which the first region 4b of the folded absorbent body 5 is joined to an intermediate position of the waist member 8 as shown in Fig. 3.

As shown in Fig. 2, the folding drum 52 which is disposed adjacent to the second unit 40 receives the wearable article continuous body 9 from the second unit 40 and conveys it. The folding drum 52 is structured similarly to the folding device disclosed in Patent Literature 2, and has a winding portion 53 that opens and closes while moving along a cylindrical conveyance route 56 and a plurality of swinging pads 54.

The winding portion 53 moves along the cylindrical conveyance route 56 while holding the parts where the absorbent bodies 4 of the waist member continuous body 6 of the wearable article continuous body 9 overlap by sucking them to a suction surface 53s where non-illustrated suction holes communicating with a negative pressure source are formed (see the partial schematic view (E-2)) and holding them.

The swinging pads 54 swing, as indicated by the arrow 54x, between an opened position P aligned with the winding portion 53 in the axial direction of the conveyance route 56 along the conveyance route 56 and a closed position Q facing the winding portion 53 in the radial direction of the conveyance route 56 with the conveyance route 56 in between. The swinging pads 54 move along the conveyance route 56 together with the winding portion 53 while holding the second regions 4f of the absorbent bodies 4 of the wearable article continuous body 9. The swinging pads 54 hold the second regions 4f of the absorbent bodies 4 of the wearable article continuous body 9 by sucking them to suction surfaces 54s where non-illustrated suction holes communicating with a negative pressure source are formed (see the partial schematic view (E-2)), and move with a predetermined spacing provided therebetween.

When the swinging pads 54 are at the opened position P, the winding portion 53 starts to hold the waist member continuous body 6 of the wearable article continuous body 9, and the swinging pads 54 start to hold the second regions 4f of the absorbent bodies 4 of the wearable article continuous body 9. While moving along the cylindrical conveyance route 56, the swinging pads 54 swing to the closed position Q to place the second regions 4f of the absorbent bodies 4 on the first regions 4b of the absorbent bodies 4, thereby folding the absorbent bodies 4 in half. On the absorbent bodies 5 folded in half, fold lines 4x (see partial schematic view (H-1)) are formed outside the waist member continuous body 6 of the wearable article continuous body 9.

After folding the absorbent bodies 4 in half, the swinging pads 54 stop holding the second regions 4f of the absorbent bodies 4 and return to the opened position P. The wearable article continuous body 9 where the absorbent bodies 4 have been folded in half is conveyed being held by the winding portion 53, and is conveyed to the non-illustrated cutter by the non-illustrated conveyer through the guide roll 58 rotated in the direction indicated by the arrow 59. As shown in Fig. 3, the non-illustrated cutter cuts the wearable article continuous body 9 at cutting positions 9x between the adjacent folded absorbent bodies 5 of the wearable article continuous body 9, thereby forming the disposable wearable articles 10 in each of which the first region 4b of the folded absorbent body 5 are joined to an intermediate position of the waist member 8.

Although not shown, as another mode, the cutter is disposed so as to face the folding drum 52, and the wearable article continuous body 9 being conveyed by the folding drum 52 is cut by the cutter. In this case, the timing when the folding drum 52 folds the absorbent bodies 4 in half may be before, after or simultaneously with the cutting of the waist member continuous body 6 of the wearable article continuous body 9.

The temporarily fixing device 60 is disposed, for example, along the folding drum 52, and temporarily fixes the first region 4b and the second region 4f of each folded absorbent body 5 together by heat sealing, ultrasonic wave sealing or the like. That is, the joining between the first region 4b and the second region 4f of each absorbent body 5 is made with a strength that can be released when the disposable wearable article 10 is used. The temporarily fixing device 60 may be structured so as to apply an adhesive to appropriate positions of the absorbent bodies 4 before being folded in half or apply double-sided tape instead of heat sealing, ultrasonic wave sealing or the like.

While the temporarily fixing device 60 may be omitted, performing temporarily fixing with the temporarily fixing device 60 being provided stabilizes the folded state of the absorbent bodies 4 and makes it possible to convey the wearable article continuous body 9 to which the folded absorbent bodies 5 are joined at higher speed, so that the number of disposable wearable articles 10 manufactured per unit time can be increased.

The size changing device 70 is structured so as to change one or both of a first conveyance speed at which the first unit 20 conveys the continuous body of the absorbent bodies 4 and a second conveyance speed at which the second unit 40 and the winding portion 53 of the third unit 50 convey the waist member continuous body 6 according to the size of the disposable wearable articles 10 and change the spacing between the absorbent bodies 5 placed on the waist member continuous body 6 at the second unit 40. For example, the size changing device 70 includes a controller that controls a servo motor driving the first unit 20, the repitching device 30, the second unit 40, the winding portion 53 of the folding drum 52 of the third unit 50 and the like, and changes the rotation speed of the servo motor according to the size of the disposable wearable articles 10.

While the size changing device 70 may be omitted, the provision of the size changing device 70 makes it possible to accommodate size differences of the disposable wearable article 10.

In the disposable wearable article manufacturing device 12, since the folding drum 52 of the third unit 50 folds the absorbent bodies 4 in half while conveying the wearable article continuous body 9 along the cylindrical conveyance route 56, the section for folding the absorbent bodies in half can be made short compared with when the absorbent bodies 4 are folded in half while the wearable article continuous body 9 is conveyed along a linear conveyance route.

Next, a method for manufacturing the disposable wearable article 10 will be described with reference to Fig. 3. As shown in Fig. 3, the disposable wearable article 10 is formed by using the absorbent body continuous body 2 and the waist member continuous body 6.

The method for manufacturing the disposable wearable article 10 is provided with the following steps (1) to (6):

### (1) First conveying step

The absorbent body continuous body 2 is conveyed in the first direction 2x indicated by the arrow 2x. In the absorbent body continuous body 2, the first sections 3 to be made into the absorbent bodies 4 are connected in the first direction 2x. The absorbent bodies 4 may be continuously formed and conveyed, or previously prepared absorbent bodies 4 may be conveyed.

### (2) Second conveying step

The waist member continuous body 6 is conveyed in the second direction 6x indicated by the arrow 6x. In the waist member continuous body 6, the second sections 7 to be made into the waist members 8 are connected in the second direction 6x. The waist member continuous body 6 may be continuously formed and conveyed, or a previously prepared waist member continuous body 6 may be conveyed.

### (3) Turning step

The absorbent bodies are turned 90 degrees. For example, the absorbent bodies 4 are conveyed in a direction indicated by the arrow 5x, and the direction of the absorbent bodies 4 relative to the conveyance direction 5x is changed by 90 degrees so that the direction connecting the ends 4p and 4q of the absorbent bodies 4 is orthogonal to the conveyance direction 5x of the absorbent bodies 4.

### (4) Joining step

The first regions 4b on the end 4p side of the 90-degree turned absorbent bodies 4 are successively placed on the waist member continuous body 6 being conveyed in the second direction 6x with a spacing provided therebetween such that the second regions 4f on the end 4q side of the absorbent bodies 4 protrude from the waist member continuous body 6, and the first regions 4b of the absorbent bodies 4 are joined to the waist member continuous body 6, thereby forming the wearable article continuous body 9 in which the first regions 4b of the absorbent bodies 4 are joined to the waist member continuous body 6.

For example, the absorbent bodies 4 and the waist member continuous body 6 are joined together by previously applying an adhesive to appropriate positions of the absorbent body 4 and/or the waist member continuous body 6. The absorbent bodies 4 and the waist member continuous body 6 may be joined together by heat sealing, ultrasonic wave sealing or the like instead of an adhesive.

### (5) Folding step

The absorbent bodies 4 are folded in half while the wearable article continuous body 9 is being conveyed. At this time, the second regions 4f of the absorbent bodies 4 are placed on the first regions 4b of the absorbent bodies 4 while the wearable article continuous body 9 is being conveyed along a cylindrical conveyance route, thereby folding the absorbent bodies 4 in half.

For example, as shown in Fig. 2, by using (a) the winding portion 53 that moves along the cylindrical conveyance route 56 and (b) the swinging pads 54 that swing between the opened position P aligned with the winding portion 53 in the axial direction of the conveyance route 56 along the conveyance route 56 and the closed position Q facing the winding portion 53 in the radial direction of the conveyance route 56 with the conveyance route 56 in between, and move along the cylindrical conveyance route 56 together with the winding portion 53, (i) when the swinging pads 54 are at the opened position P, the winding portion 53 starts to hold the waist member continuous body 6 of the wearable article continuous body 9 and the swinging pads 54 start to hold the second regions 4f of the absorbent bodies 4 of the wearable article continuous body 9, and (ii) while moving along the conveyance route 56, the swinging pads 54 swing to the closed position Q to place the second regions 4f of the absorbent bodies 4 on the first regions 4b of the absorbent bodies 4, thereby folding the absorbent bodies 4 in half.

### (6) Second cutting step

While the wearable article continuous body 9 is being conveyed, the waist member continuous body 6 of the wearable article continuous body 9 is cut at the cutting positions 9x between the adjacent absorbent bodies 4 or 5.

The order of the folding step and the second cutting step is arbitrary. For example, the second cutting step may be performed after the folding step, and the folding step may be performed after the second cutting step. The folding step and the second cutting step may be performed at the same time. The second cutting step may be started before the folding step ends, or the folding step may be started before the second cutting step ends.

By the above-described steps (1) to (6), the disposable wearable articles 10 are formed in each of which the first region 4b of the folded absorbent body 5 is joined to an intermediate position of the waist member 8.

A non-illustrated hook-and-loop fastener for joining to the second region 4f of the folded absorbent body 5 may be provided on parts 8a and 8b near both ends of the waist member 8 of the disposable wearable article 10. This hook-and-loop fastener may be previously provided on the waist member continuous body 6, or may be provided on the disposable wearable articles 10 after the cutting. Other accessory members may be previously provided on the absorbent body continuous body 2 and/or the waist member continuous body 6, or may be provided on the disposable wearable articles 10 after the cutting.

Since the absorbent bodies 4 are folded in half while the wearable article continuous body 9 to which the absorbent bodies 4 are joined is being conveyed along the cylindrical conveyance route 56, the section for folding the absorbent bodies 4 in half can be made short compared with when the absorbent bodies 4 are folded in half while the wearable article continuous body 9 to which the absorbent bodies 4 are joined is being conveyed along a linear conveyance route.

The method for manufacturing the disposable wearable article 10 may be further provided with any one or more of the following steps (7) to (9).

### (7) Temporarily fixing step

The first region 4b and the second region 4f of the folded absorbent body 5 are temporarily fixed together. For example, after the absorbent body 4 is folded in half, appropriate positions of the folded absorbent body 5 are temporarily fixed by heat sealing, ultrasonic wave sealing or the like. Alternatively, they are temporarily fixed by applying an adhesive or applying double-sided tape to appropriate positions of the absorbent body 4 before folded in half. Since the temporary fixing stabilizes the folded state, the wearable article continuous body 9 to which the folded absorbent body 5 is joined can be conveyed at higher speed, so that the number of disposable wearable articles manufactured per unit time can be increased.

### (8) Repitching step

By using the moving pad 32 (see Fig. 1), the 90-degree turned absorbent bodies 4 are received from the turning process, and the received absorbent bodies 4 are transferred to the joining step. The moving pad 32 receives the 90-degree turned absorbent bodies 4 from the turning step while moving at a first speed, and transfers the received absorbent bodies 4 to the joining step while moving at a second speed difference from the first speed. The repitching step makes it possible to change the spacing between the absorbent bodies 4 placed on the waist member continuous body 6 at the boding step.

### (9) Size changing step

According to the size of the disposable wearable articles, the spacing between the absorbent bodies 4 placed on the waist member continuous body 6 at the joining step is changed by changing one or both of the first conveyance speed at which the continuous body of the absorbent bodies 4 is conveyed at the first conveying step and the second conveyance speed at which the waist member continuous body 6 is conveyed at the second conveying step. This makes it possible to accommodate size differences of the disposable wearable articles 10 even if the sizes of the absorbent bodies 4 and/or the waist members 8 of the disposable wearable articles 10 are changed.

[Summary] As described above, by folding the absorbent bodies in half while the wearable article continuous body to which the absorbent bodies are joined is being conveyed along the cylindrical conveyance route, the section for folding the absorbent bodies in half can be shortened.

The present invention is not limited to the above-described embodiment but may be carried out with various modifications being added.

For example, the winding portion and/or the swinging pad of the third unit may hold the waist member continuous body of the wearable article continuous body and/or the second regions of the absorbent bodies by a method other than the suction by a negative pressure.

### DESCRIPTION OF REFERENCE NUMERALS

2 Absorbent body continuous body
2x First direction
3 First section
4 Absorbent body
4b First region
4f Second region
5 Folded absorbent body
6 Waist member continuous body
6x Second direction
7 Second section
8 Waist member
9 Wearable article continuous body
9x Cutting position
10 Disposable wearable article
12 Disposable wearable article manufacturing device
20 First unit
30 Repitching device
32 Moving pad
40 Second unit
50 Third unit
53 Winding portion
54 Swinging pad
56 Cylindrical conveyance route
60 Temporarily fixing device
70 Size changing device
P Opened position
Q Closed position

## Claims

1. A disposable wearable article manufacturing method comprising:
a first conveying step of conveying, in a first direction, an absorbent body continuous body in which first sections to be made into absorbent bodies are connected in the first direction;
a second conveying step of conveying, in a second direction, a waist member continuous body in which second sections to be made into waist members are connected in the second direction;
a first cutting step of forming the absorbent bodies by successively cutting the absorbent body continuous body being conveyed in the first direction;
a turning step of turning the absorbent bodies 90 degrees;
a joining step of forming a wearable article continuous body by successively placing first regions on one end side of the 90-degree turned absorbent bodies on the waist member continuous body being conveyed in the second direction with a spacing provided therebetween such that second regions on the other end side of the absorbent bodies protrude from the waist member continuous body, and joining the first regions of the absorbent bodies to the waist member continuous body;
a folding step of folding the absorbent bodies in half while the wearable article continuous body is being conveyed; and
a second cutting step of cutting the waist member continuous body of the wearable article continuous body at cutting positions between the adjacent absorbent bodies while the wearable article continuous body is being conveyed,
wherein at the folding step, the absorbent bodies are folded in half by placing the second regions of the absorbent bodies on the first regions of the absorbent bodies while the wearable article continuous body is being conveyed along a cylindrical conveyance route.

2. The disposable wearable article manufacturing method according to claim 1, further comprising
a temporarily fixing step of temporarily fixing the first regions and the second regions of the folded absorbent bodies together.

3. The disposable wearable article manufacturing method according to claim 1 or 2, further comprising
a repitching step of, by using a moving pad, receiving the 90-degree turned absorbent bodies from the turning step, and transferring the received absorbent bodies to the joining step,
the moving pad receiving the 90-degree turned absorbent bodies from the turning step while moving at a first speed, and transferring the received absorbent bodies to the joining step while moving at a second speed different from the first speed.

4. The disposable wearable article manufacturing method according to claim 1 or 2, further comprising
a size changing step of changing, according to the size of the disposable wearable articles, the spacing between the absorbent bodies placed on the waist member continuous body at the joining step by changing one or both of a first conveyance speed at which the absorbent body continuous body is conveyed at the first conveying step and a second conveyance speed at which the waist member continuous body is conveyed at the second conveyance step.

5. A disposable wearable article manufacturing device comprising:
a first unit that forms absorbent bodies by cutting an absorbent body continuous body in which first sections to be made into the absorbent bodies are connected in a first direction while conveying the absorbent body continuous body in the first direction, and turns the absorbent bodies 90 degrees;
a second unit that forms a wearable article continuous body by conveying, in a second direction, a waist member continuous body in which second sections to be made into waist members are connected in the second direction, successively placing first regions on one end side of the 90-degree turned absorbent bodies on the waist member continuous body being conveyed with a spacing provided therebetween such that second regions on the other end side of the absorbent bodies protrude from the waist member continuous body, and joining the first regions of the absorbent bodies to the waist member continuous body; and
a third unit that folds the absorbent bodies of the wearable article continuous body in half and cuts the wearable article continuous body at cutting positions between the adjacent absorbent bodies to form disposable wearable articles in each of which the first region of the folded absorbent body is joined to an intermediate position of the waist member,
wherein the third unit has:
a winding portion that moves along a cylindrical conveyance route; and
a swinging pad that swings between an opened position adjacent to the winding portion in an axial direction of the conveyance route along the conveyance route and a closed position facing the winding portion in a radial direction of the conveyance route with the conveyance route in between, and moves along the conveyance route together with the winding portion,
when the swinging pad is at the opened position, the winding portion starts to hold the waist member continuous body of the wearable article continuous body and the swinging pad starts to hold the second region of the absorbent body of the wearable article continuous body, and
while moving along the conveyance route, the swinging pad swings to the closed position to place the second region of the absorbent body on the first region of the absorbent body, thereby folding the absorbent body in half.

6. The disposable wearable article manufacturing device according to claim 5, further comprising
a temporarily fixing device that temporarily fixes the first regions and the second regions of the folded absorbent bodies together.

7. The disposable wearable article manufacturing device according to claim 5 or 6, further comprising
a repitching device having a moving pad that receives the 90-degree turned absorbent bodies from the first unit while moving at a first speed and transfers the received absorbent bodies to the second unit while moving at a second speed different from the first speed.

8. The disposable wearable article manufacturing device according to claim 5 or 6, further comprising
a size changing device that changes, according to the size of the disposable wearable articles, the spacing between the absorbent bodies placed on the waist member continuous body by changing one or both of a first conveyance speed at which the first unit conveys the absorbent body continuous body and a second conveyance speed at which the second unit and the winding portion of the third unit convey the waist member continuous body.

## Patentansprüche

1. Ein Verfahren zur Herstellung tragbarer Einwegartikel, umfassend:
einen ersten Förderschritt, bei dem in einer ersten Richtung ein kontinuierlicher Absorptionskörper gefördert wird, in dem erste Abschnitte, aus denen Absorptionskörper hergestellt werden sollen, in der ersten Richtung verbunden sind;
einen zweiten Förderschritt, bei dem in einer zweiten Richtung ein durchgehender Taillenelementkörper gefördert wird, in dem in der zweiten Richtung zweite Abschnitte verbunden sind, aus denen Taillenelemente hergestellt werden sollen;
einen ersten Schneidschritt zum Formen der Absorptionskörper durch sukzessives Schneiden des Absorptionskörpers, wobei der kontinuierliche Körper in die erste Richtung befördert wird;
ein Drehschritt, bei dem die Absorptionskörper um 90 Grad gedreht werden;
einen Verbindungsschritt zum Bilden eines kontinuierlichen Körpers für tragbare Artikel durch sukzessives Platzieren erster Bereiche auf einer Endseite der um 90 Grad gedrehten Absorptionskörper auf dem kontinuierlichen Körper des Taillenelements, der in die zweite Richtung befördert wird, mit einem Abstand dazwischen, so dass zweite Bereiche auf der anderen Endseite der Absorptionskörper aus dem kontinuierlichen Körper des Taillenelements herausragen, und Verbinden der ersten Bereiche der Absorptionskörper mit dem durchgehenden Körper des Taillenelements;
einen Faltschritt, bei dem die Absorptionskörper in zwei Hälften gefaltet werden, während der kontinuierliche Körper des tragbaren Artikels transportiert wird; und
Ein zweiter Schneidschritt des Schneidens des kontinuierlichen Taillenelementkörpers des kontinuierlichen Körpers des tragbaren Artikels an Schneidpositionen zwischen den benachbarten absorbierenden Körpern, während der kontinuierliche Körper des tragbaren Artikels transportiert wird
wobei beim Faltschritt die Absorptionskörper in zwei Hälften gefaltet werden, indem die zweiten Bereiche der Absorptionskörper auf den ersten Bereichen der Absorptionskörper platziert werden, während der kontinuierliche Körper des tragbaren Artikels entlang einer zylindrischen Förderroute transportiert wird.

2. Das Verfahren zur Herstellung tragbarer Einwegartikel nach Anspruch 1, das weiterhin umfasst
Ein vorübergehender Fixierungsschritt zum vorübergehenden Fixieren der ersten Bereiche und der zweiten Bereiche der gefalteten Absorptionskörper zusammen.

3. Verfahren zur Herstellung tragbarer Einwegartikel nach Anspruch 1 oder 2, weiterhin umfassend
einen Umschaltschritt, bei dem unter Verwendung eines beweglichen Pads die um 90 Grad gedrehten Absorptionskörper aus dem Drehschritt aufgenommen und die aufgenommenen Absorptionskörper in den Verbindungsschritt überführt werden
Das bewegliche Pad nimmt die um 90 Grad gedrehten Absorptionskörper aus dem Drehschritt auf, während es sich mit einer ersten Geschwindigkeit bewegt, und überträgt die aufgenommenen Absorptionskörper zum Verbindungsschritt, während es sich mit einer zweiten Geschwindigkeit bewegt, die sich von der ersten Geschwindigkeit unterscheidet.

4. Verfahren zur Herstellung tragbarer Einwegartikel nach Anspruch I oder 2, weiterhin umfassend
ein Größenänderungsschritt zum Ändern, entsprechend der Größe der tragbaren Einwegartikel, den Abstand zwischen den Absorptionskörpern, die beim Verbindungsschritt auf dem kontinuierlichen Körper des Taillenelements platziert werden, durch Ändern einer ersten Fördergeschwindigkeit, mit der der kontinuierliche Körper des Absorptionskörpers beim ersten Förderschritt gefördert wird, und einer zweiten Fördergeschwindigkeit, mit der der kontinuierliche Körper des Taillenelements beim zweiten Förderschritt gefördert wird, oder beider.

5. Eine Vorrichtung zur Herstellung tragbarer Einwegartikel, umfassend:
eine erste Einheit, die Absorptionskörper bildet, indem sie einen kontinuierlichen Absorptionskörper schneidet, wobei die ersten Abschnitte, aus denen die Absorptionskörper hergestellt werden sollen, in einer ersten Richtung verbunden sind, während der kontinuierliche Absorptionskörper in die erste Richtung befördert wird, und die Absorptionskörper um 90 Grad dreht;
eine zweite Einheit, die einen durchgehenden Körper für tragbare Gegenstände bildet, indem sie in einer zweiten Richtung einen durchgehenden Körper für Taillenelemente transportiert, in dem zweite Abschnitte, die zu Taillenelementen verarbeitet werden sollen, in der zweiten Richtung verbunden sind, sukzessives Platzieren erster Bereiche auf einer Endseite der um 90 Grad gedrehten Absorptionskörper auf dem durchgehenden Taillenelementkörper, der mit einem dazwischen vorgesehenen Abstand transportiert wird, so dass zweite Bereiche auf der anderen Endseite der Absorptionskörper aus dem durchgehenden Taillenelementkörper herausragen, und Verbinden der ersten Bereiche der Absorptionskörper mit dem durchgehenden Taillenelementkörper; und
eine dritte Einheit, die die Absorptionskörper des durchgehenden Körpers des tragbaren Artikels in zwei Hälften faltet und den durchgehenden Körper des tragbaren Artikels an Schneidpositionen zwischen den benachbarten Absorptionskörpern schneidet, um tragbare Einwegartikel zu bilden, bei denen der erste Bereich des gefalteten Absorptionskörpers jeweils mit einer Zwischenposition des Taillenelements verbunden ist,
wobei die dritte Einheit Folgendes aufweist:
einen Wickelabschnitt, der sich entlang einer zylindrischen Förderstrecke bewegt; und
ein Schwingpolster, das zwischen einer geöffneten Position neben dem Wickelabschnitt in axialer Richtung der Förderstrecke entlang der Förderstrecke und einer geschlossenen Position, die dem Wickelabschnitt in radialer Richtung der Förderstrecke zugewandt ist, mit der Förderstrecke dazwischen schwingt und sich zusammen mit dem Wickelabschnitt entlang der Förderstrecke bewegt,
Wenn sich das Schwingpolster in der geöffneten Position befindet, beginnt der Wickelabschnitt, den durchgehenden Taillenelementkörper des durchgehenden Körpers des tragbaren Artikels zu halten, und das Schwingpolster beginnt, den zweiten Bereich des absorbierenden Körpers des durchgehenden Körpers des tragbaren Artikels zu halten, und
Während der Bewegung entlang der Förderstrecke schwingt das Schwingpolster in die geschlossene Position, um den zweiten Bereich des Absorptionskörpers auf dem ersten Bereich des Absorptionskörpers zu platzieren und dadurch den Absorptionskörper in zwei Hälften zu falten.

6. Vorrichtung zur Herstellung tragbarer Einwegartikel nach Anspruch 5, weiterhin umfassend
Eine temporäre Befestigungsvorrichtung, die die ersten Bereiche und die zweiten Bereiche der gefalteten Absorptionskörper vorübergehend aneinander fixiert.

7. Vorrichtung zur Herstellung tragbarer Einwegartikel nach Anspruch 5 oder 6, weiterhin umfassend
eine Repitching-Vorrichtung mit einem beweglichen Pad, das die um 90 Grad gedrehten Absorptionskörper von der ersten Einheit aufnimmt, während es sich mit einer ersten Geschwindigkeit bewegt, und die aufgenommenen Absorptionskörper an die zweite Einheit überträgt, während es sich mit einer zweiten Geschwindigkeit bewegt, die sich von der ersten Geschwindigkeit unterscheidet.

8. Vorrichtung zur Herstellung tragbarer Einwegartikel nach Anspruch 5 oder 6, weiterhin umfassend
eine Größenänderungsvorrichtung, die sich je nach Größe der tragbaren Einwegartikel ändert, den Abstand zwischen den auf dem kontinuierlichen Körper des Taillenelements platzierten Absorptionskörpern durch Ändern einer ersten Fördergeschwindigkeit, mit der die erste Einheit den kontinuierlichen Körper des Absorptionskörpers befördert, oder einer zweiten Fördergeschwindigkeit, mit der die zweite Einheit und der Wickelabschnitt der dritten Einheit den kontinuierlichen Körper des Taillenelements befördern.

## Revendications

1. Procédé de fabrication d'articles portables jetables comprenant :
une première étape de transport consistant à transporter, dans une première direction, un corps absorbant continu dans lequel des premières sections à transformer en corps absorbants sont connectées dans la première direction ;
une deuxième étape de transport consistant à transporter, dans une deuxième direction, un corps continu d'élément de taille dans lequel des deuxièmes sections à transformer en éléments de taille sont reliées dans la deuxième direction ;
une première étape de découpe consistant à former les corps absorbants en découpant successivement le corps absorbant, le corps continu étant transporté dans la première direction ;
une étape de rotation consistant à tourner les corps absorbants à 90 degrés ;
une étape de jonction consistant à former un corps continu d'article portable en plaçant successivement des premières régions sur un côté d'extrémité des corps absorbants tournés à 90 degrés sur le corps continu de l'élément de taille transporté dans la seconde direction avec un espacement prévu entre elles de telle sorte que les secondes régions sur l'autre côté d'extrémité des corps absorbants dépassent du corps continu de l'élément de taille, et joindre les premières régions des corps absorbants au corps continu de l'élément de taille ;
une étape de pliage consistant à plier les corps absorbants en deux pendant que le corps continu de l'article portable est transporté ; et
une deuxième étape de coupe consistant à couper le corps continu de l'élément de taille du corps continu de l'article portable à des positions de coupe entre les corps absorbants adjacents pendant que le corps continu de l'article portable est transporté,
dans lequel, lors de l'étape de pliage, les corps absorbants sont pliés en deux en plaçant les secondes régions des corps absorbants sur les premières régions des corps absorbants tandis que le corps continu de l'article portable est transporté le long d'un trajet de transport cylindrique.

2. Procédé de fabrication d'articles portables jetables selon la revendication 1, comprenant en outre
une étape de fixation temporaire consistant à fixer temporairement ensemble les premières régions et les secondes régions des corps absorbants pliés.

3. Procédé de fabrication d'articles portables jetables selon la revendication 1 ou 2, comprenant en outre une étape de repitchage consistant, à l'aide d'un tampon mobile, à recevoir les corps absorbants tournés à 90 degrés de l'étape de tournage et à transférer les corps absorbants reçus à l'étape de jonction,
le tampon mobile recevant les corps absorbants tournés à 90 degrés de l'étape de rotation tout en se déplaçant à une première vitesse, et transférant les corps absorbants reçus à l'étape de jonction tout en se déplaçant à une seconde vitesse différente de la première vitesse.

4. Procédé de fabrication d'articles portables jetables selon la revendication 1 ou 2, comprenant en outre une étape de changement de taille consistant à modifier, en fonction de la taille des articles portables jetables, l'espacement entre les corps absorbants placés sur le corps continu de l'élément de taille à l'étape de jonction en modifiant l'une ou les deux d'une première vitesse de transport à laquelle le corps continu du corps absorbant est transporté à la première étape de transport et d'une seconde vitesse de transport à laquelle le corps continu de l'élément de taille est transporté à la seconde étape de transport.

5. Dispositif de fabrication d'articles portables jetables comprenant :
une première unité qui forme des corps absorbants en coupant un corps absorbant continu dans lequel les premières sections à transformer en corps absorbants sont connectées dans une première direction tout en transportant le corps absorbant continu dans la première direction, et fait tourner les corps absorbants de 90 degrés ;
une seconde unité qui forme un corps continu d'article portable en transportant, dans une seconde direction, un corps continu d'élément de taille dans lequel des secondes sections à transformer en éléments de taille sont reliées dans la seconde direction, placer successivement des premières régions sur un côté d'extrémité des corps absorbants tournés à 90 degrés sur le corps continu de l'élément de taille transporté avec un espacement prévu entre eux de telle sorte que des secondes régions sur l'autre côté d'extrémité des corps absorbants dépassent du corps continu de l'élément de taille, et joindre les premières régions des corps absorbants au corps continu de l'élément de taille ; et
une troisième unité qui plie les corps absorbants du corps continu de l'article portable en deux et coupe le corps continu de l'article portable à des positions de coupe entre les corps absorbants adjacents pour former des articles portables jetables dans chacun desquels la première région du corps absorbant plié est jointe à une position intermédiaire de l'élément de taille,
dans lequel la troisième unité a :
une partie d'enroulement qui se déplace le long d'un trajet de transport cylindrique ; et
un tampon oscillant qui oscille entre une position ouverte adjacente à la partie d'enroulement dans une direction axiale du trajet de transport le long du trajet de transport et une position fermée faisant face à la partie d'enroulement dans une direction radiale du trajet de transport avec le trajet de transport entre les deux, et se déplace le long du trajet de transport avec la partie d'enroulement,
lorsque le tampon oscillant est en position ouverte, la partie d'enroulement commence à maintenir le corps continu de l'élément de taille du corps continu de l'article portable et le tampon oscillant commence à maintenir la seconde région du corps absorbant du corps continu de l'article portable, et
tout en se déplaçant le long du trajet de transport, le tampon oscillant oscille vers la position fermée pour placer la deuxième région du corps absorbant sur la première région du corps absorbant, pliant ainsi le corps absorbant en deux.

6. Dispositif de fabrication d'articles portables jetables selon la revendication 5, comprenant en outre
un dispositif de fixation temporaire qui fixe temporairement ensemble les premières régions et les secondes régions des corps absorbants pliés.

7. Dispositif de fabrication d'articles portables jetables selon la revendication 5 ou 6, comprenant en outre
un dispositif de repitching comportant un tampon mobile qui reçoit les corps absorbants tournés à 90 degrés de la première unité tout en se déplaçant à une première vitesse et transfère les corps absorbants reçus à la seconde unité tout en se déplaçant à une seconde vitesse différente de la première vitesse.

8. Dispositif de fabrication d'articles portables jetables selon la revendication 5 ou 6, comprenant en outre
un dispositif de changement de taille qui change, en fonction de la taille des articles portables jetables, l'espacement entre les corps absorbants placés sur le corps continu de l'élément de taille en modifiant l'une ou les deux d'une première vitesse de transport à laquelle la première unité transporte le corps continu du corps absorbant et d'une seconde vitesse de transport à laquelle la seconde unité et la partie d'enroulement de la troisième unité transportent le corps continu de l'élément de taille.
